# EUROPEAN PATENT SPECIFICATION

(11) **EP 2 866 555 B1**
(45) Date of publication and mention of the grant of the patent: **14.11.2018**
(21) Application number: 13806385.4
(22) Date of filing: 21.06.2013
(51) Int. Cl.: A01N 25/00, A61K 31/365, A61K 31/215, A61K 31/4184, A61K 31/17, A61P 33/00, A61P 33/02, A61P 33/10, A61P 33/14, F42B 12/00, A61D 7/00

(54) **DOSAGE REGIME FOR ANIMAL TREATMENT**
DOSIERUNGSSCHEMA ZUR BEHANDLUNG VON TIEREN
RÉGIME POSOLOGIQUE POUR LE TRAITEMENT D'UN ANIMAL

(30) Priority: 22.06.2012 AU 2012902640; 11.04.2013 AU 2013204030
(43) Date of publication of application: 06.05.2015
(73) Proprietor: Smartvet Pty Ltd, Fig Tree Pocket, QLD 4069 (AU)
(72) Inventor: WEYER, Grant, Fig Tree Pocket QLD 4069 (AU)
(74) Representative: Crease, Devanand John
(86) International application number: PCT/AU2013/000673
(87) International publication number: WO 2013/188928

(56) References cited:
- WO-A1-2008/052263
- WO-A2-2005/074672
- WO-A2-2006/057658
- US-A- 3 528 662
- US-A1- 2004 089 186
- US-B1- 6 524 286
- MOLENTO M.B.: 'Parasite control in the age of drug resistance and changing agricultural practices' VETERINARY PARASITOLOGY vol. 163, no. 3, 2009, pages 229 - 234, XP026266859
- ELANCO: 'StandGuard Pour-on Insecticide, product information' DRUGS.COM May 2007, XP055181163 Retrieved from the Internet: <URL:http://www.durvet.com/index.php%3Fopti on%3Dcom_k2%26view%3Ditem%26task% 3Ddownload%26id%3D1509 3c3270cee54b93ec862fe946c5e5ef47&sa=U&ei=ox LV UdzPCaiOiQfMmYCoBA&ved=OCBsQFjAA&usg=AFQjCN GcneLptB7h5eMPRhBIsjLhaYPq5w> [retrieved on 2013-07-04]

## Description

### Technical Field

The present invention relates to dosage regimes and methods of treating animals suitable for reducing the likelihood of a target pest developing pesticide resistance.

### Background

Animal pests, particularly in commercially important animals such as cattle, are typically controlled using chemical pesticides. A common problem with this approach is that the pest will often develop resistance to a chemical pesticide after repeated exposure of a population of pests to the same pesticide. Thus, repeated use of the same pesticide is often ineffective in controlling pests. Consequently, pesticide resistance can result in a decline in an animal's vigour and general health.

Currently, the problem of pesticide resistance is addressed by annually rotating the classes of chemical compounds used to control a target pest over multiple pest seasons. In this way the target pest is not repeatedly exposed to compounds of the same chemical class, thereby aiming to prevent the pest from developing pesticide resistance.

In a commercial setting the producer is responsible for the choice of pesticide and its administration to each animal to be treated. Accordingly, it is the producer who chooses which pesticide is to be used every year. The selection of the most appropriate compounds or classes of compounds for annual rotation requires an understanding of pesticide chemistry that many producers do not possess.

In addition, pesticides are marketed under brand names and the same pesticide or class of pesticide may be marketed under multiple brand names. Because of this it is often unclear to producers to which chemical class a particular pesticide belongs. This increases the difficulty for the producer of selecting a pesticide of a different class each year. These difficulties often, cause producers to avoid rotating the chemical classes used and simply continuing to use a pesticide which has previously been successful. This practice can accelerate the development of pesticide resistance.

Molento M.B. (Veterinary Parasitology, vol. 163, no.3, 2009, pages 229-234, XP026266859) discusses the problem of parasite resistance in livestock and solutions that have been proposed to address this problem, including slow drug rotation where drugs are alternated on a yearly basis. Elanco: "StandGuard Pour-on Insecticide, product information"(drugs.com, May 2007, XP055181163) discloses applications of a spinosyn at the beginning and end of a pest season, and the conditional use of a pyrethroid if the incidence of a pest is a above a threshold. WO 2008/052263 discloses projectiles for administration of insecticides.

The present inventors have developed a dosage regime to assist producers to treat animal pests and reduce the likelihood of the development of pesticide resistance. The dosage regime may be used to reduce existing pesticide resistance or delay the development of pesticide resistance.

### Summary of Invention

In a first aspect the invention provides a dosage regime for use in the treatment of an animal for a pest over a pest season, the dosage regime comprising:
a first dosage projectile having a unique identifier and containing a first pesticide;
a second dosage projectile having a unique identifier and containing a second pesticide; and
a third dosage projectile having a unique identifier and containing a third pesticide;
wherein the dosage projectiles are configured for remotely delivering the pesticides to an animal; and
wherein the unique identifiers indicate the timing of administration of each pesticide over a pest season.

The animal may be ovine, bovine, equine, leporine, porcine, feline, canine, caprine or avian. Preferably, the animal is bovine.

The pest may be a horn fly, face fly, stable fly, heel fly, warble fly, bot fly, house fly, horse fly, deer fly, blow fly, mosquito, midge, louse, tick, and mite. The target pest may be a helminth such as a roundworm, stomach worm, tapeworm, or fluke. The target pest may be a protozoan such as a coccidian. In some embodiments the target pest may be a larvae, pupae or egg.

Preferably at least one of the dosage projectiles is a substantially non-skin piercing dosage projectile. More preferably, the first, second and third dosage projectiles are substantially non-skin piercing dosage projectiles.

Preferably, the projectile is adapted to deliver the pesticide to the animal substantially without piercing the skin of the animal.

The shell of the projectile may be made of any suitable encapsulating material, such as, for example, gelatine, linear polymers, plastics, waxes and hardeners such as carnauba, candelilla, bees, paraffin, stearic acid, synthetic polymers, polyesters, polylactic acid, starch copolymers, high molecular weight polyvinylalcohol, un-stabilized polyethelyne, un-stabilized polypropylene, biodegradable polymers, compostable polymers, biopolymers or polystyrene derivatives, thin-walled plastics materials, continuous outer coatings or layers of a polymerizable liquid, hydrophilic colloidal materials such as, gelatin, albumin, gum arabic, alginate, casein, agar or pectins, or combinations thereof, or synthetic organic compounds such as, but not limited to, polystyrene, polypropylene, polyethylene, polycarbonate, polyamide, polysulfane, polyvinylchloride, resinous compounds such as fibreglass or poly(methyl methacrylate) (eg Perspex) derivatives, or combinations thereof.

Preferably, the frangible shell of the projectile is made of soft gelatine, glycerol and/or sorbitol and combinations thereof.

The pesticide may be a macrocyclic lactone such as an avermectin or milbemycin, including but not limited to ivermectin, eprinomectin, moxidectin, selamectin, doramectin, milbemycin oxime, abamectin, and emamectin benzoate.

The pesticide may be a synthetic pyrethroid such as flumethrin, permethrin, deltamethrin, cypermethrin, cyfluthrin, lambda cyhalothrin, gamma cyhalothrin fenvalerate, alphacypermethrin and pyrethrin.

The pesticide may be an insect growth regulator such as pyriproxifen, methoprene, cyromazine, lufenuron, diflubenzuron, fluazuron, dicyclanil and fluazuron.

The pesticide may be a pro-insecticide being a compound that is metabolized into an active insecticide after entering the host or target insect. The pro-insecticide may be derived from a microbially produced compounds for example halogenated pyrroles, an example of this class being chlorfenapyr.

The pesticide may be an anthelminitic such as fipronil, imidacloprid, rotenone, magnesium flurosilicate, piperonyl butoxide, or a spinosyn.

The pesticide may be a benzimidazole such as albendazole, oxfenbendazole, fenbendazole.

The pesticide may be an immunomodulator, for example levamisole.

In one preferred regime, the first projectile contains for example a synthetic pyrethroid, the second projectile contains for example a macrocyclic lactone and the third projectile contains for example an insect growth regulator.

The pesticide may be encapsulated or micro-encapsulated in one or more encapsulating or coating agents in order to more effectively control the delivery of the pesticide to the animal. The encapsulating or coating agent may be chosen such that it regulates the release of the pesticide once it has been deposited onto the skin or absorbed into the blood stream or lymphatic system of the animal.

The dosage projectile may further include or be delivered in conjunction with a marker. The marker may be a non-toxic, physiologically acceptable dye, sufficient to mark the skin, coat or fur of an animal for a period of about 1 hour to about 21 days, preferably about 1 to 7 days. The marker may be a pharmaceutical, food, or cosmetic colorant, oil based paint or colorant, pigment, or dye. Examples of suitable marking components include liquid dyes, powder dyes, water soluble dyes, infra-red dyes, ultraviolet dyes, or dyes that glow in the dark (eg chemiluminescent dyes or phosphorescent dyes). It is to be understood that the dyes may be selected so as not to impair or interfere with the action or efficacy of the pesticide.

The marker may be fluorescent, rendering animals which have been marked or treated under low-light conditions easily visible. The marker and/or solution may also contain a radioactive or other suitable tracking component.

In one preferred form, the dosage projectile further includes a transdermal carrier as defined in WO 2008/052263 assigned to Smartvet Pty Ltd. In this form, the pesticide may be transported (with the aid of a transdermal agent) through the skin following rupturing of the projectile upon contact with the animal, penetration of the pesticide being effected by the transdermal carrier contained within the projectile. In use the transdermal carrier facilitates passage of the pesticide through the skin of an animal in need of treatment.

It is to be understood that any suitable transdermal carrier or solvent which facilitates transdermal absorption of the pesticide may be used. Typically, the transdermal carrier includes carriers such as isopropyl alcohol, dipropylene glycol methyl-ether, butylated hydroxytoluene dipropylene glycol monomethyl-ether, 1-methoxy 2-propanol (glysolv PM/Icinol PM), Ethylene glycol monobutylether (butyl glyxolv/butyl icinol), Butyl di glysolv (butyl-icinol), Transcutol, propylene glycol (PG), N-methyl-2 pyrrolidone (NMP), methylene chloride, diethyl ether, ethanol, acetonitrile, ethyl acetate, benzyl alcohol and a combination of natural oils; ethylene glycol, propylene glycol, dimethyl polysiloxane (DMPX), oleic acid, caprylic acid, 1-octanol, ethanol (denatured or anhydrous), liposomal compositions, suitable plant oils, such as *Aloe vera* derivatives or sesame seed oil or derivatives thereof, acrylic polymers, rubber-based polymers, polysiloxane-based polymers, polyvinylpyrrolidone-based polymers, dimethylsulfoxide (DMSO), dimethylformamide (DMF), lecithin, Transfersomes® (IDEA AG). Transfersomes® are artificial vesicles designed to mimic a cell vesicle and deliver drugs or genetic material into a cell. The bounding membrane of a Transfersomes® is more flexible than that of a liposome, allowing it to deform and pass through openings in a barrier, such as the skin, whose diameters are much smaller than the average vesicle size. A Transfersome® is a bi-component, most often vesicular, aggregate. The main functional characteristic of the aggregate is the extreme flexibility and permeability of its bilayer-like membrane coating. The basis of this characteristic is the interdependency of local membrane shape and composition, which makes the bilayer self-regulating and self-optimising. The bilayer is thus capable of stress adaptation, via local and reversible bilayer component demixing. These characteristics make Transfersomes® a device suitable for non-invasive and targeted drug delivery, including across intact skin.

Additional transdermal carriers include, but are not limited to, ethosomes, azone, castor oil derivatives, such as ethoxylated castor oil, jojoba oil derivatives, corn oil derivatives, emu oil derivatives, or any other suitable transdermal or transcutaneous carrier or carrier composition.

Preferably, the transdermal carrier is propylene glycol, DMSO, alcohol or a more penetrant adjuvant known to the art.

In one embodiment the unique identifier is a symbol, code, marking, colour, or feature of a dosage projectile. The unique identifier may be a word or symbol printed on the surface of the dosage projectile.

In a preferred embodiment the identifier is a colour or patch of colour on the dosage projectile. The colour may be incorporated into the shell of the projectile or the colour may be a dye contained within the projectile visible to the naked eye.

In one embodiment the first, second and third unique identifiers indicate that each pesticide is to be administered at a predetermined time in a pest season. For example, the first dosage projectile having a first unique identifier may indicate administration of the first pesticide at the beginning of the pest season. The second dosage projectile having a unique identifier may indicate administration of the second pesticide at the midpoint of the pest season, and the third dosage projectile having a unique identifier may indicate administration of the third pesticide at or near the end of the pest season.

The pesticide in the dosage projectile may be remotely delivered using a launcher, such as an compressed-air launcher.

Typically, the dosage projectile is shot from a launching device such as a gun, pressure or gas activated launcher or the like. Examples of suitable launching devices may be based on similar gas discharge technologies utilized in known dart guns, air guns, crowd control guns and paintball markers currently in production and used in the veterinary, security, law enforcement, hunting, and recreational shooting or paintball industry.

The dosage projectiles may be launched with a single trigger action from a projectile launcher. The projectile launcher may include a selector for selecting the number of projectiles to be launched with a single trigger action. Alternatively, the projectiles may be delivered using a semi-automatic trigger action.

The dosage projectiles, when used to deliver multiple pesticides or doses, may include pesticides which are similar or differing in composition, efficacy, or pharmaceutical action. Accordingly, it is to be understood that the dose administered to an animal may be controlled by selecting the number and type of dosage forms or dosage projectiles to be launched at an animal. In this way, a user can easily adjust the dose required for correctly dosing the animal, by compensating for the size and weight of an animal, and tailor dosing regimens.

It will be appreciated that the treatment may be prophylactic.

The projectile launcher may include velocity selection means operable to select the velocity at which the projectile is launched. For example the velocity selection means may include pressure-regulating means operable to select the pressure at which the pressurized fluid is released.

The launching propellant may be a pressurized fluid, such as gas or air.

The dosage regime may further comprise at least a further dosage projectile having a unique identifier and containing a further pesticide.

The disclosure also provides a method for treating an animal over a pest season, the method comprising:
remotely administering to an animal a series of different pesticides over a pest season using a dosage regime according to the first aspect of the present invention, wherein timing of administration of the pesticides is carried out in accordance with the unique identifier of the dosage projectiles.

Preferably, the method comprises:
remotely administering a first dosage projectile having a unique identifier and containing a first pesticide early in a pest season;
remotely administering a second dosage projectile having a unique identifier and containing a second pesticide around mid pest season; and
remotely administering a third dosage projectile having a unique identifier and containing a third pesticide late in a pest season;
wherein timing of administration of the pesticides is carried out in accordance with the unique identifier.

Preferably, the method utilizes a combination of pesticides with each having a different mode of activity, thereby assisting in reducing the likelihood of a target pest developing pesticide resistance to any one particular pesticide. In some examples the method may be used to reduce existing pesticide resistance or delay development of pesticide resistance in a pest or population of pests.

The disclosure additionally provides a kit for treating an animal over a pest season, the kit comprising:
a dosage regime comprising a first dosage projectile having a unique identifier and containing a first pesticide; a second dosage projectile having a unique identifier and containing a second pesticide; and a third dosage projectile having a unique identifier and containing a third pesticide;
wherein the dosage projectiles are configured for remotely delivering the pesticides to an animal and the unique identifiers indicate the timing of administration of each pesticide; and
instructions for administering each pesticide in accordance with the unique identifiers.

### Definitions

As used herein the term 'pest season' refers to the period of time that a pest colonises, infects or is transiently, periodically or continually associated with an animal.

As used herein, the term 'pest' refers to any organism or microorganism that negatively affects an animal by colonisation, infection or transient, periodic or continual association with the animal. The term 'pest' includes organisms that spread disease and/or compete with an animal for nutrients. In addition, a pest is any organism or microorganism known to associate with an animal and which, as a result of that association, causes a detrimental effect on the animal's health and vigour. The term 'pest' refers to an individual pest organism or microorganism or a population of pest organisms or microorganisms.

As used herein, the term 'pesticide' refers to any substance that can be used in the control of a pest, that is, a pesticide is any substance that can prevent association of a pest with an animal, destroy or repel a pest, or mitigate the effect of a pest on an animal. The term 'pesticide' also refers to naturally occurring and synthetic chemicals, and chemicals synthetically derived from naturally occurring compounds.

As used herein the term 'pesticide resistance' refers to the acquired ability of a pest or population of pests to survive in the presence of, or at a certain concentration of, a pesticide which is normally effective in preventing association of the pest or population of pests with an animal, destroying or repelling a pest, or mitigating the effect of a pest or population of pests on an animal.

As used herein the term 'dosage projectile' includes a substantially non-skin piercing comprising a frangible shell and capable of containing a pesticide within the shell.

As used herein the term 'remote delivery' refers to any method of delivering a pesticide to an animal which does not require immediate proximity to the animal.

As used herein the term 'identifier' refers to any symbol, marking, or feature of a dosage projectile. In particular embodiments the 'identifier' may be a colour.

Unless the context requires otherwise or specifically stated to the contrary, integers, steps, or elements of the invention recited herein as singular integers, steps or elements clearly encompass both singular and plural forms of the recited integers, steps or elements.

The term 'animal' is to be accorded its widest meaning, and includes humans and individuals of any species of social, economic or research importance including but not limited to members of the genus ovine, bovine, equine, porcine, feline, canine, primates (including human and non-human primates), rodents, murine, caprine, leporine, and avian. In preferred embodiments the animal is a bovine animal.

Those skilled in the art will appreciate that the invention described herein is susceptible to variations and modifications other than those specifically described. It is to be understood that the invention includes all such variations and modifications that fall within the scope of the appended claims.

Throughout this specification, unless the context requires otherwise, the word 'comprise', or variations such as 'comprises' or 'comprising', will be understood to imply the inclusion of a stated element, integer or step, or group of elements, integers or steps, but not the exclusion of any other element, integer or step, or group of elements, integers or steps.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed before the priority date of each claim of this specification.

### Detailed Description

In accordance with the present invention, dosage regimes are provided for reducing the likelihood of a target pest developing pesticide resistance. The disclosure also provides methods and kits for reducing the likelihood of a target pest developing pesticide resistance. The dosage regimes and methods generally include administering to an animal a series of different pesticides over a pest season using dosage projectiles coded to indicate the timing of administration of each pesticide.

### Methods and Dosage Regimes

The dosage regimes and methods disclosed aim to reduce the likelihood of a target pest developing pesticide resistance by administering a series of different pesticides to an animal within a single pest season.

The administration of a series of different pesticides is typically used to sequentially administer or rotate the administration of pesticides, each with a different mode of activity. In some examples this results in a regression of pesticide resistance or a delay in the development of pesticide resistance.

The dosage regimes and methods employ the remote delivery of a series of pesticides using dosage projectiles each containing a different pesticide. Typically, for each animal to be treated three different dosage projectiles are used, each containing a different pesticide. In some examples the method may comprise at least a further dosage projectile having a further unique identifier and a further pesticide. In other examples it is envisaged that a further 1, 2, 3, 4, 5, 6, 7, or 8 further dosage projectiles each having a further unique identifier and a further pesticide may be used.

Each dosage projectile has a unique identifier. It is contemplated that the identifier may be any symbol, marking, or feature of a dosage projectile. For example the identifiers may be words or symbols printed on the surface of the dosage projectiles. In another embodiment the dosage projectile is manufactured with a raised or recessed symbols or words on the surface of the projectile.

In a preferred embodiment the identifier is a colour or patch of colour on the dosage projectile. In some embodiments the colour is printed or painted onto the dosage projectile. In other embodiments the dosage projectile may be manufactured with a coloured surface or with a colour incorporated into the shell of the projectile.

The colour is typically a dye. In some embodiments the dye may be mixed with the contents of the dosage projectile.

Typically, the dosage projectiles are used to remotely deliver the pesticides to the animal. In a preferred example remote delivery is achieved by launching the projectiles from a projectile launcher. Suitable launchers are known in the art. For example WO 2008/052263 discloses a preferred projectile launcher for use in the present invention. Typically, a single trigger action releases a single projectile. The projectile launcher may include a selector for selecting the number of projectiles to be launched with a single trigger action. Alternatively, the projectiles may be delivered using a semi-automatic trigger action.

Accordingly, it is to be understood that the dose administered to an animal may be controlled by selecting the number and type of dosage forms or projectiles to be launched at an animal. In this way, a user can easily adjust the dose required for correctly dosing the animal, by compensating for the size and weight of an animal, and tailor dosing regimens.

It will be appreciated that an animal may be prophylactically treated using the method of the invention to reduce the likelihood of a target pest developing pesticide resistance.

The dosage regimes and methods use unique identifiers of each dosage projectile to indicate the timing of administration of each pesticide during the pest season.

The timing is predetermined by the nature of the target pest and can be determined by a skilled person with a knowledge of the target pest. For example adult horn flies often first emerge in early spring and will immediately feed from cattle and then lay eggs in fresh droppings. After the eggs hatch and the larvae have pupated more adults typically emerge in 10 to 20 days. This results in large numbers of horn flies in early summer. A second peak in fly numbers usually occurs in late summer or early autumn.

The dosage regimes and methods of the present disclosure, when used to reduce the likelihood of horn flies developing pesticide resistance may involve administering different pesticide in each of early spring, early summer and then late summer or early autumn. That is, each pesticide may be administered at times of high pest numbers. Alternatively, the pesticides may be administered prior to the expected times of high pest numbers.

It is contemplated that any, or any combination of, symbols, colours or features of the dosage projectiles may be used as unique identifiers. By way of example, the first, second and third dosage projectiles may each be coloured red, yellow and green. In this example, the red projectile may contain a synthetic pyrethroid, the yellow projectile may contain a macrocylic lactone, and the green projectile may contain a halogenated pyrrole such as chlorfenapyr.

In use the projectiles are supplied with instructions indicating that, for example the red projectile is to be administered in early spring or a time corresponding to the emergence of flies, the yellow projectiles are to be used in early summer or a time corresponding to a first peak in fly numbers, and the green projectiles are to be used in late summer or early autumn or a time corresponding to a second peak in horn fly numbers.

### Pests

The dosage regimes and methods of the present invention are useful in reducing the likelihood of any target pest or population of target pests developing pesticide resistance.

The target pest may be an insect horn fly, face fly, stable fly, heel fly, warble fly, bot fly, house fly, horse fly, deer fly, blow fly, mosquito, midge, flea or louse.

The target pest may be an arachnid such as a tick or mite.

The target pest may be a helminth such as a roundworm, stomach worm, tapeworm, or trematoda such as a flatworm or fluke.

The target pest may be a protozoan such as a coccidian.

In some embodiments the target pest may be an adult pest, a larvae, pupae, egg or any combination thereof.

### Dosage Projectiles

The dosage projectiles used in the invention are of the type known in the art. In a preferred embodiment the dosage projectiles marketed under the name VetCap® as described in WO 2008/052263 are used in the methods and dosage regimens of the invention. It should be noted that the present invention does not require the use of a transdermal agent with the pesticides but a transdermal agent may optionally be employed for treating certain pests.

Typically, dosage projectiles used in the present invention are made from a substance such as, but not limited to, hydrophilic colloidal materials such as, gelatin, albumin, gum arabic, alginate, casein, agar or pectins, or combinations thereof. The projectile can also be made from a synthetic organic compound such as, but not limited to, plastics, synthetic polymers, polyesters, polylactic acid, starch copolymers, high molecular weight polyvinylalcohol, un-stabilized polyethelyne, un-stabilized polypropylene, biodegradable polymers, compostable polymers, biopolymers (including those comprised of polylactic acid), polystyrene, polypropylene, polyethylene, polycarbonate, polyamide, polysulfane, polyvinylchloride, resinous compounds such as fibreglass or Perspex derivatives or combinations thereof, waxes and hardeners such as carnauba, candelilla, bees, paraffin, stearic acid, or combinations thereof.

The dosage projectile includes at least one pesticide. The pesticide can be encapsulated in a controlled-release coating prior to inclusion in the dosage projectile thereby allowing the controlled release of the pesticide within an animal to be treated animal, once it has passed transdermally into the blood or lymphatic system of the animal. The controlled-release coating may be selected from controlled release compositions known in the field.

Preferably the viscosity of the projectile contents should be such that the contents do not run off the skin, fur or coat of the animal prematurely before treatment has occurred. Accordingly, the projectile may also include a thickening agent, such as a starch-like compound, inert polymer, gel, or an oil-based composition such as sesame seed oil, if required.

The pesticides contained in the projectile can be in different forms and/or concentrations, depending on the formulation, the carrying capacity, and solubility and release characteristics desired, for example as neutral molecules, components of molecular complexes, and pharmaceutically acceptable salts, free acids or bases, or quaternary, salts thereof. Simple derivatives of the pesticides mentioned herein, such as pharmaceutically acceptable ethers, esters, amides and the like which have desirable retention and release characteristics in vivo, and enzymes, pro-active forms, pro-drugs and the like, can also be employed as required.

The dosage projectiles may include additional components to enhance the effectiveness of the pesticides or to reduce discomfort to an animal.

In some examples the dosage projectile may include a transdermal carrier. The term 'transdermal carrier' or refers to any material known in the art as being suitable for transdermal pesticide administration, and includes any polymeric material into which a pesticide may be solubilised in combination or admixture with the other ingredients to form a composition. The term also includes enhancers, solvents, co-solvents, carriers and other types of additives useful for facilitating transdermal pesticide delivery, or adhesives for ensuring adhesion of the contents of the projectile to the skin, coat or fur of a target animal.

The amount of pesticide to be complexed with the transdermal carrier will vary depending on the particular pesticide, and the time span for which the pesticide effective. Normally, the amount of pesticide in the transdermal system can vary from about 0.1% to about 50%, or even from about 0.1 % to about 30% by weight based on the dry weight of the total carrier composition. Persons skilled in the field of the invention will be able to determine the adequate amounts required for each application, as required.

It is to be appreciated that the order of steps, the amounts of the ingredients, and the amount and time of mixing may be important process variables which will depend on the specific polymers, pesticides, solvents and/or co-solvents, enhancers, additives and/or excipients used in the composition.

The transdermal carrier, if used, is typically used in an amount of about 1% to about 95%, and preferably from about 10% to about 75%, by weight based on the weight of the total carrier composition.

The transdermal carrier composition can also contain one or more solvents and/or co-solvents known in the art.

Suitable solvents and co-solvents include volatile substances or compositions such as alcohols, aromatic hydrocarbons such as benzene derivatives, lower molecular weight alkanes and cycloalkanes, alkanoic acid esters, polyhydric alcohols, which include glycols, triols and polyols such as ethylene glycol, diethylene glycol, propylene glycol, dipropylene glycol, trimethylene glycol, butylene glycol, polyethylene glycol, hexylene glycol, polyoxethylene, glycerin, trimethylpropane, sorbitol, polyvinylpyrrolidone, glycol ethers such as ethylene glycol monoethyl ether, glycol esters, glycol ether esters such as ethylene glycol monoethyl ether acetate and ethylene glycol diacetate; saturated and unsaturated fatty acids, mineral oil, silicone fluid, lecithin, retinol derivatives and the like, and ethers, esters and alcohols of fatty acids, or combinations and mixtures thereof.

Although the exact amount of solvents and co-solvents that may be used in the carrier composition depends on the nature and amount of the other ingredients, such amount typically ranges from about 0.1% to about 50%, and preferably from about 0.1% to about 30% by weight, and more preferably from about 1% to about 20%, by weight based on the dry weight of the total carrier composition.

The transdermal carrier is typically selected so that it may be readily absorbable by the skin of an animal without causing undue itching, irritation, or toxic effects to the animal. Selection of the transdermal carrier will also depend on the pesticide to be delivered to an animal and also the type of animal to be treated, or the intended delivery site on an animal. Thus, the transdermal carrier composition may be selected to suit the charge, size, hydrophobicity, hydrophilicity, amphipathicity, pl, pH, decay rate, or other relevant criteria of the pesticide to be carried transdermally, while also being readily absorbable through the skin of an animal.

Typically, the transdermal carrier includes compounds such as isopropyl alcohol, dipropylene glycol methyl-ether, butylated hydroxytoluene dipropylene glycol monomethyl-ether, methylene chloride, 1-methoxy 2-propanol (glysolv PM/Icinol PM), Ethylene glycol monobutylether (butyl glyxolv/butyl icinol), Butyl di glysolv (butyl-icinol), Transcutol, propylene glycol (PG), N-methyl-2 pyrrolidone (NMP), diethyl ether, ethanol, acetonitrile, ethyl acetate, benzyl alcohol and a combination of natural oils. ethylene glycol, propylene glycol, dimethyl polysiloxane (DMPX), oleic acid, caprylic acid, 1-octanol, ethanol (denatured or anhydrous), liposomal compositions, suitable plant oils, such as *Aloe vera* derivatives or sesame seed oil or derivatives thereof, acrylic polymers, rubber-based polymers, polysiloxane-based polymers, polyvinylpyrrolidone-based polymers, dimethylsulfoxide (DMSO), dimethylformamide (DMF), lecithin, Transfersomes®, ethosomes, azone, castor oil derivatives, such as ethoxylated castor oil, jojoba oil derivatives, corn oil derivatives, emu oil derivatives, or other suitable carriers.

In certain examples of the invention, an enhancer is incorporated into the carrier composition. The term 'enhancers' as used herein refers to substances used to increase permeability and/or accelerate the delivery of an active agent through the skin of an animal, and include monohydric alcohols such as ethyl, isopropyl, butyl and benzyl alcohols; or dihydric alcohols such as ethylene glycol, diethylene glycol, or propylene glycol dipropylene glycol and trimethylene glycol; or polyhydric alcohols such as glycerin, sorbitol and polyethylene glycol, which enhance drug solubility; polyethylene glycol ethers of aliphatic alcohols (such as cetyl, lauryl, oleyl and stearly) including polyoxyethylene-4-lauryl ether, polyoxyethylene-2-oleyl ether and polyoxyethylene-10-oleyl ether; vegetable, animal and fish fats and oils such as cotton seed, corn, safflower, olive and castor oils, squalene, and lanolin; fatty acid esters such as propyl oleate, decyl oleate, isopropyl palmitate, glycol palmitate, glycol laurate, dodecyl myristate, isopropyl myristate and glycol stearate which enhance drug diffusibility; fatty acid alcohols such as oleyl alcohol and its derivatives; fatty acid amides such as oleamide and its derivatives; urea and urea derivatives such as allantoin which affect the ability of keratin to retain moisture; polar solvents such as dimethyldecylphosphoxide, methyloctylsulfoxide, dimethyllaurylamide, dodecylpyrrolidone, isosorbitol, dimethylacetonide, dimethylsulfoxide, decylmethylsulfoxide and dimethylformamide; salicylic acid; benzyl nicotinate; or higher molecular weight aliphatic surfactants such as lauryl sulfate salts, esters of sorbitol and sorbitol anhydride such as polysorbate. Other suitable enhancers include oleic and linoleic acids, triacetin, ascorbic acid, panthenol, butylated hydroxytoluene, tocopherol, tocopherol acetate, tocopheryl linoleate.

If enhancers are incorporated into the carrier composition, the amount typically ranges up to about 35%, and preferably from about 0.05% to about 20%, by weight based on the dry weight of the total carrier composition.

### Unique Identifier

The dosage projectiles used in the present invention comprise a unique identifier. It is contemplated that a unique identifier may be any symbol, marking, or feature of a dosage projectile. For example the identifiers may be words or symbols printed on the surface of the dosage projectiles. In another embodiment the dosage projectile is manufactured with a raised or recessed symbols or words on the surface of the projectile.

In a preferred embodiment the identifier is a colour or patch of colour on the dosage projectile. In some embodiments the colour is printed or painted onto the dosage projectile. In other embodiments the dosage projectile may be manufactured with a coloured surface or with a colour incorporated into the shell of the projectile.

The unique identifier may be a pharmaceutically acceptable dye or marker composition. The dye or marker is released onto the skin of an animal when the projectile ruptures upon impact with the animal. The dye or marker may be brightly coloured to allow a person administering the treatment to see readily which animals have been treated, and where the projectile has ruptured on the animal. The dye or marker is preferably non-permanent in nature, so that it may no longer be visible on treated animals within a day or two.

In one example, the dye is a fluorescent dye such that animals, especially nocturnal animals, may be marked or treated at night time.

The dyes used in rupturable projectiles currently used in paintball games are generally considered suitable for use in the projectiles of the invention (and are available in a wide variety of colours) provided they do not interfere with the pesticide or the transdermal carrier included in the projectile. In a preferred embodiment the principal dye colours used would be red, yellow and green FD&C dyes. FD & C dyes are the dyes certified and allowed in the United States for the Food, Pharmaceutical, Cosmetics & Personal Care industry. FD &C dyes include but are not limited to the dyes set out in Table 1:

**Table 1: Examples of FD & C dyes**

| **FD & C No** | **Colour Index No** | **EEC No** | **Colour Index reference (Food)** |
|---|---|---|---|
| FD & C Red 40 | 16035 | E 129 | Red 17 |
| FD & C Yellow 5 | 19140 | E 102 | Yellow 4 |
| FD & C Yellow 6 | 15985 | E 110 | Yellow 3 |
| FD & C Blue 1 | 42090 | E 133 | Blue 2 |
| FD & C Blue 2 | 73015 | E 132 | Blue 1 |
| FD & C Red 3 | 45430 | E 127 | Red 14 |
| FD & C Green 3 | 42053 | - | Green 3 |

### Remote delivery

The dosage projectiles of the dosage regime of the invention are configured for remotely delivering the pesticides. This is typically achieved using a projectile launcher, for example an air launcher. The projectile launcher can include a magazine or reservoir for accepting a plurality of projectiles. Administering a pesticide to an animal is accomplished by a person or user aiming the launcher containing one or more projectiles at the animal, and launching a projectile at the animal with a velocity sufficient to rupture the projectile upon impact with the animal. This allows the contents of the projectile to be splattered onto the skin of the animal, allowing the pesticide to be absorbed through the skin of the animal via the transdermal carrier. The impact of the projectile against the animal also serves to splatter a marker dye, if present, onto the skin of the animal, thereby enabling the user administering the treatment to readily discern whether the animal has been treated, where the site of impact was on the animal (eg if the site of impact is important to the efficacy and absorption of a specific pesticide) and whether the projectile has ruptured successfully or not.

The dosage projectiles may have sufficient volume to contain a unit dosage for a certain pest of an animal. The dosage is typically calculated to correspond to a certain minimum weight of animal to which a pesticide is to be administered. If larger animals are to be treated, the number of projectiles per animal may be increased accordingly. Alternatively, a single projectile dosage for all animal weights may be preserved by alteration of the formulation concentration of the active pharmaceutical agent.

The launcher can have a selector button which allows a user to pre-select the number of projectiles to be launched at the single pull of a trigger of the launcher, thereby allowing larger animals to be treated with the correct dose required, merely by selecting the number of projectiles to be launched substantially simultaneously. This has the advantage that the animal does not have a chance to escape following the first firing of the launcher, as the projectiles reach it substantially simultaneously. Launching one projectile at a time may result in the animal fleeing, making it difficult to track down the same animal and administer a second (or different) dose.

Similarly, it may be necessary to treat an animal with a combination of pesticides. This may be accomplished by using a projectile containing a combination of pesticides. However, it is not always possible to produce a projectile having two or more different pesticides therein, due to adverse reactions occurring between such pesticides when they are co-mixed.

### Pesticides

It is contemplated that any pesticide which is deliverable to an animal to produce a desired, usually beneficial, effect may be used in the dosage regimes of the present invention. It should be noted that the pesticides can be used singularly or in combinations or mixtures as required.

Examples of pesticides useful in the present invention include parasiticides and/or anthelmintics which include, but are not limited to the following:

Macrocyclic lactones including the avermectins and milbemycins, for example Ivermectin, eprinomectin, moxidectin, selamectin, doramectin, milbemycin, abamectin, cydectin and emamectin benzoate.

Synthetic pyrethroids such as flumethrin, deltamethrin, cypermethrin, cyfluthrin, lambda cyhalothrin, fenvalerate, alphacypermethrin and pyrethrin.

Insect growth regulators such as pyriproxifen, methoprene, cyromazine, lufenuron, diflubenzuron, fluazuron, dicyclanil and fluazuron.

Anthelminitics such as fipronil, imidacloprid, rotenone, magnesium flurosilicate, piperonyl butoxide, spinosyns and other suitable benzimidazole anthelmintics and immunomodulators (e.g. Levamisole).

Anthelmintic, anti-trematodal, anticestodal, or anti-parasitic/parasiticidal agents such as albendazole, levamisole, mebendazole, pyrantel, praziquantel, moxidectin, ivermectin, oxamniquine, metrifonate, piperazine, thiabendazole, tiabendazole, diethylcarbamazine, pyrantel, niclosamide, doramectin, eprinomectin, morantel, oxfendazole, dichlorvos, chlorsulon and selamectin.

The pesticide may be a pro-insecticide being a compound that is metabolized into an active insecticide after entering the host or target insect. The pro-insecticide may be derived from a microbially produced compounds for example halogenated pyrroles, an example of this class being chlorfenapyr.

Acaricidal agents including antibiotic acaricides, nikkomycins, thuringiensin, macrocyclic lactones, acaricides, tetranactin, avermectin, acaricides, abamectin, doramectin, eprinomectin, ivermectin, selamectin, milbemycin, acaricides, milbemectin, milbemycin oxime, moxidectin, bridged diphenyl acaricides, azobenzene, benzoximate, benzyl benzoate, bromopropylate, chlorbenside, chlorfenethol, chlorfenson, chlorfensulphide, chlorobenzilate, chloropropylate, cyflumetofen, dicofol, diphenyl sulfone, dofenapyn, fenson, fentrifanil, fluorbenside, proclonol, tetradifon, tetrasul, carbamate acaricides, benomyl, carbanolate, carbaryl, carbofuran, methiocarb, metolcarb, promacyl, propoxur, oxime carbamate caricides, aldicarb, butocarboxim, oxamyl, thiocarboxime, thiofanox, dinitrophenol acaricides, binapacryl, dinex, dinobuton, dinocap, dinocap-6, dinocton, dinopenton, dinosulfon, dinoterbon, DNOC, formamidine acaricides, amidines, amitraz, chlordimeform, chloromebuform, formetanate, formparanate, mite growth regulators, clofentezine, diflovidazin, dofenapyn, fluazuron, flubenzimine, flucycloxuron, flufenoxuron, hexythiazox, organochlorine acaricides, bromocyclen, camphechlor, dienochlor, endosulfan, lindane, organophosphorus acaricides, organophosphate acaricides, chlorfenvinphos, crotoxyphos, dichlorvos, heptenophos, mevinphos, monocrotophos, TEPP, tetrachlorvinphos, organothiophosphate acaricides, amidithion, amiton, azinphos-ethyl, azinphos-methyl, azothoate, benoxafos, bromophos, bromophos-ethyl, carbophenothion, chlorpyrifos, chlorthiophos, coumaphos, cyanthoate, demeton, demeton-O, demeton-S, demeton-methyl, demeton-O-methyl, demeton-S-methyl, demeton-S-methylsulphon, dialifos, diazinon, dimethoate, dioxathion, disulfoton, endothion, ethion, ethoate-methyl, formothion, malathion, mecarbam, methacrifos, omethoate, oxydeprofos, oxydisulfoton, parathion, phenkapton, phorate, phosalone, phosmet, phoxim, pirimiphos-methyl, prothidathion, prothoate, pyrimitate, quinalphos, quintiofos, sophamide, sulfotep, thiometon, triazophos, trifenofos, vamidothion, phosphonate acaricides, trichlorfon, phosphoramidothioate acaricides, isocarbophos, methamidophos, propetamphos, phosphorodiamide caricides, dimefox, mipafox, schradan, organotin acaricides, azocyclotin, cyhexatin, fenbutatin, phenylsulfamide acaricides, dichlofluanid, phthalimide acaricides, dialifos, phosmet, pyrazole acaricides, acetoprole, fipronil, tebufenpyrad, vaniliprole, pyrethroid acaricides, pyrethroid ester caricides, acrinathrin, bifenthrin, cyhalothrin, cypermethrin, alpha-ypermethrin, fenpropathrin, fenvalerate, flucythrinate, flumethrin, fluvalinate, tau-fluvalinate, permethrin, pyrethroid ether acaricides, halfenprox, pyrimidinamine acaricides, pyrimidifen, pyrrole acaricides, chlorfenapyr, quinoxaline acaricides, chinomethionat, thioquinox, sulfite ester caricides, propargite, tetrazine acaricides, clofentezine, diflovidazin, tetronic acid acaricides, spirodiclofen, thiocarbamate acaricides, fenothiocarb, thiourea acaricides, chloromethiuron, diafenthiuron, unclassified acaricides, acequinocyl, amidoflumet, arsenous oxide, bifenazate, closantel, crotamiton, disulfiram, etoxazole, fenazaflor, fenazaquin, fenpyroximate, fluacrypyrim, fluenetil, mesulfen, MNAF, nifluridide, pyridaben, sulfiram, sulfluramid, sulfur triarathene.

Cholinergic agents such as acetylcholine, arecoline, bethanechol, carbachol, choline, methacoline, muscarine and pilocarpine.

Anti-cholinergic agents such as atropine, eucatropine and procyclidine.

The amounts of the pesticide to be used in each dosage projectile may be determined by methods known to persons skilled in the art. Amounts typically range from about 0.05 mg to about 20,000 mg, and preferably from about 0.1 mg to about 1,000 mg, depending on the pesticide, the target, the animal species, the size of the animal. In certain embodiments of the invention, the pesticides may be included in a range from about 0.1 to about 500 mg per mammal per 50 kg body weight.

Although the illustrative embodiments of the present invention have been described, it is to be understood that the invention is not limited to those precise embodiments, and that various other changes and/or modifications may be made by one skilled in the art without departing from the scope of the invention as defined in the appended claims.

The examples provided herein are not to be interpreted as being an exhaustive list of possible integers or embodiments of the invention, and serve merely to illustrate the invention.

The present invention will now be further described in greater detail by reference to the following specific example, which should not be construed as in any way limiting the scope of the invention as defined in the appended claims.

### EXAMPLE

A red coloured dosage projectile is provided. The red dosage projectile is composed of principally a gelatine and sorbitol shell, and containing a payload of the synthetic pyrethroid lambda cyhalothrin at 1.5% synergised with 7.5% piperonyl butoxide and using corn oil as the main excipient. This dosage projectile was administered to each cow in a given herd during the Spring season, as soon as horn fly burdens exceed the economic threshold of 200 flies per side.

Between 2 and 8 weeks later (depending the level of resistance exhibited in the given fly population), a yellow similarly constituted dosage projectile, containing the macrocyclic lactone ivermectin at 3%, in microemulsion with isopropanol, is administered.

Between a further 2 and 8 weeks after administration of the yellow dose, a green capsule containing the main active ingredient chlorfenapyr solubilised in a proprietary SmartVet solvent base is administered.

Current 'best practice' encourages the rotation of chemical classes in successive annual seasons, but the lack of a simple and convenient way to select the optimal chemical combinations, has impeded successful implementation. The treatment program detailed herein, demonstrates the unique ability of the dosage capsules used in combination with VetCap™ delivery system, to efficiently facilitate the rapid rotation of active molecules within the same season, with the payload of each pesticide in each capsule pre-selected for its different mode of activity. Pre-selection of the active molecule in the yellow capsule is done in such a way to promote the progeny of any flies which survive, and successfully breed, after exposure to the first 'red' dosage capsule, being sensitised to exposure to the second chemical class in the 'yellow' dosage capsule. If any flies exhibit resistance to the yellow dosage projectile dose, their progeny are then exposed to the specially selected third chemical class in the green dosage projectile. Pre-selection of the active molecule in the green capsule is done such to promote that the progeny of any flies which survive and successfully breed after exposure to the second 'yellow' capsule dose, are sensitised to exposure to the third chemical class in the 'green' dosage capsule. In this way the rapid rotation of chemical classes serves to amplify the lethality of each subsequent pesticide used. Because of the rapid breeding cycle of flies, performing this rotation in the same season, increases the chance of each succeeding generation of flies facing distinctly different chemical challenges in rapid succession. This has the effect of dramatically interrupting the ability of the flies to adapt to the combination of chemical challenges, and thereby break the cycle of resistance development. In addition, there are indications that flies which have acquired to resistance to one specific active compound, become more susceptible to other chemical compounds. These indications have been seen in synthetic pyrethroids followed in rapid succession by dosing with a macrocyclic lactone and followed by another dose of synthetic pyrethroid. Similar indications of resistance regression has been witnessed with synthetic pyrethroid resistant flies after being exposed to chlorfenapyr, and then again exposed to a synthetic pyrethroid (hereinafter called the 'sensitisation effect'). The dosage regimes and methods of treating animals detailed herein, seek to uniquely combine the ease of delivery, intuitive colour coded rotation method and the sensitisation effect, to reduce the likelihood of a target pest developing pesticide resistance; and in many instances even regress the levels of acquired resistance/immunity that may have already developed.

It will be appreciated by persons skilled in the art that numerous variations and/or modifications may be made to the invention as shown in the specific embodiments without departing from the scope of the invention as defined by the appended claims. The present embodiments are, therefore, to be considered in all respects as illustrative and not restrictive.

## Claims

1. A dosage regime for use in the treatment of an animal for a pest over a pest season, the dosage regime comprising:
a first dosage projectile having a unique identifier and containing a first pesticide;
a second dosage projectile having a unique identifier and containing a second pesticide; and
a third dosage projectile having a unique identifier and containing a third pesticide;
wherein the dosage projectiles are configured for remotely delivering the pesticides to an animal; and
wherein the unique identifiers indicate the timing of administration of each pesticide over a pest season.

2. The dosage regime for use according to claim 1 wherein the animal is an ovine, bovine, equine, leporine, porcine, feline, canine, caprine or avian, preferably the animal is bovine.

3. The dosage regime for use according to claim 1 or 2 wherein the pest is selected from the group consisting of horn fly, face fly, stable fly, heel fly, warble fly, bot fly, house fly, horse fly, deer fly, blow fly, mosquito, midge, louse, tick, mite, protozoan and helminth, preferably the helminth is selected from the group consisting of roundworm, stomach worm, tapeworm, and fluke.

4. The dosage regime for use according to claim 3 wherein the pest is a larvae, pupae or egg.

5. The dosage regime for use according to any one of claims 1 to 4 wherein at least one of the dosage projectiles is a substantially non-skin piercing dosage projectile, preferably the first, second and third dosage projectiles are substantially non-skin piercing dosage projectiles.

6. The dosage regime for use according to any one of claims 1 to 5 wherein the first, second, and third pesticides are selected from the group consisting of macrocyclic lactones, synthetic pyrethroids, insect growth regulators, antihelminitics, benzimidazoles, and pro-insecticides, wherein at least two of the first, second, and third pesticides are not the same.

7. The dosage regime for use according to any one of claims 1 to 6 wherein the first dosage projectile contains a synthetic pyrethroid, the second dosage projectile contains a macrocyclic lactone, and the third dosage projectile contains an insect growth regulator.

8. The dosage regime for use according to claim 6 or 7 wherein the macrocyclic lactone is selected from the group consisting of avermectin, milbemycin, ivermectin, eprinomectin, moxidectin, selamectin, doramectin, milbemycin oxime, abamectin, emamectin benzoate, and a combination thereof.

9. The dosage regime for use according to claim 6 or 7 wherein the synthetic pyrethroid is selected from the group consisting of flumethrin, permethrin, deltamethrin, cypermethrin, cyfluthrin, lambda cyhalothrin, gamma cyhalothrin fenvalerate, alphacypermethrin, pyrethrin, and a combination thereof.

10. The dosage regime for use according to claim 6 or 7 wherein the insect growth regulator is selected from the group consisting of pyriproxifen, methoprene, cyromazine, lufenuron, diflubenzuron, dicyclanil, fluazuron, and a combination thereof.

11. The dosage regime for use according to claim 6 wherein the anthelminitic is selected from the group consisting of levamisole, clorsulon, fipronil, imidacloprid, rotenone, magnesium flurosilicate, piperonyl butoxide, spinosyn, and a combination thereof.

12. The dosage regime for use according to claim 6 wherein the benzimidazole is selected from the group consisting of albendazole, oxfenbendazole, and fenbendazole.

13. The dosage regime for use according to any one of claims 1 to 12 wherein the unique identifier is a symbol, code, marking, colour, or feature of a dosage projectile, preferably the unique identifier is a different sign, word, symbol or colour on each dosage projectile.

14. The dosage regime for use according to claim 13 wherein the unique identifier is a different colour on each dosage projectile, preferably the colour is incorporated into the shell of the dosage projectile or the colour is a dye contained within the projectile visible to the naked eye.

15. The dosage regime for use according to any one of claims 1 to 14, further comprising instructions for administering each pesticide in accordance with the unique identifier of each dosage projectile.

## Patentansprüche

1. Dosierungsschema zur Verwendung bei der Behandlung eines Tieres für einen Schädling während einer Schädlingssaison, wobei das Dosierungsschema Folgendes umfasst:
ein erstes Dosierungsgeschoss mit einem eindeutigen Identifikator, das ein erstes Pestizid enthält;
ein zweites Dosierungsgeschoss mit einem eindeutigen Identifikator, das ein zweites Pestizid enthält; und
ein drittes Dosierungsgeschoss mit einem eindeutigen Identifikator, das ein drittes Pestizid enthält;
wobei die Dosiergeschosse so konfiguriert sind, dass sie die Pestizide aus der Ferne an ein Tier abgeben; und
wobei die eindeutigen Identifikatoren den Zeitpunkt der Verabreichung jedes Pestizids über eine Schädlingssaison angeben.

2. Dosierungsschema zur Verwendung nach Anspruch 1, wobei das Tier ein Schaf, Rind, Pferd, Leporin, Schwein, Katze, Hund, Ziege oder Vogel ist, vorzugsweise das Tier ein Rind ist.

3. Dosierungsschema zur Verwendung nach Anspruch 1 oder 2, wobei der Schädling ausgewählt ist aus der Gruppe bestehend aus Hornfliege, Gesichtsfliege, Stallfliege, Fersenfliege, Warzenfliege, Botfliege, Stubenfliege, Pferdefliege, Hirschfliege, Blasfliege, Moskito, Mücke, Laus, Zecke, Milbe, Protozoen und Helminthe, wobei der Helminthe vorzugsweise ausgewählt ist aus der Gruppe bestehend aus Spulwurm, Magenwurm, Bandwurm und Flunder.

4. Dosierungsschema zur Verwendung nach Anspruch 3, wobei der Schädling eine Larve, eine Puppe oder ein Ei ist.

5. Dosierungsschema zur Verwendung nach einem der Ansprüche 1 bis 4, wobei mindestens eines der Dosierungsgeschosse ein im Wesentlichen nicht hautdurchdringendes Dosierungsgeschoss ist, vorzugsweise das erste, zweite und dritte Dosierungsgeschoss im Wesentlichen nicht hautdurchdringende Dosierungsgeschosse sind.

6. Dosierungsschema zur Verwendung nach einem der Ansprüche 1 bis 5, wobei die ersten, zweiten und dritten Pestizide ausgewählt sind aus der Gruppe bestehend aus makrozyklischen Lactonen, synthetischen Pyrethroiden, Insektenwachstumsregulatoren, Antihelminitika, Benzimidazolen und Proinsektiziden, wobei mindestens zwei der ersten, zweiten und dritten Pestizide nicht gleich sind.

7. Dosierungsschema zur Verwendung nach einem der Ansprüche 1 bis 6, wobei das erste Dosierungsgeschoss ein synthetisches Pyrethroid enthält, das zweite Dosierungsgeschoss ein makrozyklisches Lacton enthält, und das dritte Dosierungsgeschoss einen Insektenwachstumsregulator enthält.

8. Dosierungsschema zur Verwendung nach Anspruch 6 oder 7, wobei das makrocyclische Lacton ausgewählt ist aus der Gruppe bestehend aus Avermectin, Milbemycin, Ivermectin, Eprinomectin, Moxidectin, Selamectin, Doramectin, Milbemycinoxim, Abamectin, Emamectinbenzoat und einer Kombination davon.

9. Dosierungsschema zur Verwendung nach Anspruch 6 oder 7, wobei das synthetische Pyrethroid ausgewählt ist aus der Gruppe bestehend aus Flumethrin, Permethrin, Deltamethrin, Cypermethrin, Cyfluthrin, Lambda-Cyhalothrin, Gamma-Cyhalothrin-Fenvalerat, Alphacypermethrin, Pyrethrin und einer Kombination davon.

10. Dosierungsschema zur Verwendung nach Anspruch 6 oder 7, wobei der Insektenwachstumsregulator ausgewählt ist aus der Gruppe bestehend aus Pyriproxifen, Methopren, Cyromazin, Lufenuron, Diflubenzuron, Dicyclanil, Fluazuron und einer Kombination davon.

11. Dosierungsschema zur Verwendung nach Anspruch 6, wobei das Anthelminit aus der Gruppe bestehend aus Levamisol, Clorsulon, Fipronil, Imidacloprid, Rotenon, Magnesiumfluorsilikat, Piperonylbutoxid, Spinosyn und einer Kombination davon ausgewählt ist.

12. Dosierungsschema zur Verwendung nach Anspruch 6, wobei das Benzimidazol ausgewählt ist aus der Gruppe bestehend aus Albendazol, Oxfenbendazol und Fenbendazol.

13. Dosierungsschema für die Verwendung nach einem der Ansprüche 1 bis 12, wobei der eindeutige Identifikator ein Symbol, ein Code, eine Markierung, eine Farbe oder ein Merkmal eines Dosiergeschosses ist, wobei vorzugsweise der eindeutige Identifikator ein anderes Zeichen, Wort, Symbol oder eine andere Farbe auf jedem Dosiergeschoss ist.

14. Dosierungsschema zur Verwendung nach Anspruch 13, wobei der eindeutige Identifikator eine andere Farbe auf jedem Dosierungsgeschoss ist, wobei vorzugsweise die Farbe in die Hülle des Dosierungsgeschosses eingearbeitet ist oder die Farbe ein mit bloßem Auge sichtbarer Farbstoff innerhalb des Geschosses ist.

15. Dosierungsschema für die Verwendung nach einem der Ansprüche 1 bis 14, ferner umfassend Anweisungen für das Verabreichen jedes Pestizids in Übereinstimmung mit dem eindeutigen Identifikator jedes Dosierungsgeschosses.

## Revendications

1. Régime posologique destiné à une utilisation dans le traitement d'un animal contre un nuisible pendant une saison d'infestation, le régime posologique comprenant :
un premier projectile de dosage ayant un identificateur unique et contenant un premier pesticide ;
un deuxième projectile de dosage ayant un identificateur unique et contenant un deuxième acide ; et
un troisième projectile de dosage ayant un identificateur unique et contenant un troisième pesticide ;
les projectiles de dosage étant conçus pour administrer à distance les pesticides à un animal ; et
les identificateurs uniques indiquant le rythme d'administration de chaque pesticide pendant d'une saison d'infestation.

2. Régime posologique selon la revendication 1, dans lequel l'animal concerné est un ovin, un bovin, un équin, un léporidé, un porcin, un félin, un canin, un caprin ou un aviaire, de préférence l'animal étant un bovin.

3. Régime posologique selon la revendication 1 ou 2, dans lequel le nuisible concerné est choisi dans l'ensemble constitué de la mouche des cornes, de la mouche faciale, de la mouche des étables, de la mouche des talons, de l'hypoderme, de la gastrophile, de la mouche domestique, du taon à chevaux, du taon à cerfs, de la mouche bleue, du moustique, du moucheron, du pou, de la tique, de la mite, du protozoaire et de l'helminthe, de préférence l'helminthe étant choisi dans l'ensemble constitué du nématode, de l'hémonchus, du ténia et de la douve.

4. Régime posologique selon la revendication 3, dans lequel le nuisible concerné est une larve, une nymphe ou un oeuf.

5. Régime posologique selon l'une quelconque des revendications 1 à 4, dans lequel au moins un des projectiles de dosage est un projectile de dosage ne perçant sensiblement pas la peau, de préférence les premier, deuxième et troisième projectiles de dosage sont des projectiles de dosage ne perçant sensiblement pas la peau.

6. Régime posologique selon l'une quelconque des revendications 1 à 5, dans lequel les premier, deuxième et troisième pesticides sont choisis dans l'ensemble constitué des lactones macrocycliques, des pyréthroïdes synthétiques, des régulateurs de croissance des insectes, des antihelminitiques, des benzimidazoles et des pro-insecticides, au moins deux des premier, deuxième et troisième pesticides n'étant pas les mêmes.

7. Régime posologique selon l'une quelconque des revendications 1 à 6, dans lequel le premier projectile de dosage contient un pyréthroïde synthétique, le deuxième projectile de dosage contient une lactone macrocyclique et le troisième projectile de dosage contient un régulateur de croissance des insectes.

8. Régime posologique selon la revendication 6 ou 7, dans lequel la lactone macrocyclique est choisie dans l'ensemble constitué de l'avermectine, de la milbémycine, de l'ivermectine, de l'éprinomectine, de la moxidectine, de la sélamectine, de la doramectine, de l'oxime de milbémycine, de l'abamectine, du benzoate d'émamectine et de leurs combinaisons.

9. Régime posologique selon la revendication 6 ou 7, dans lequel le pyréthroïde synthétique est choisi dans l'ensemble constitué de la fluméthrine, de la perméthrine, de la deltaméthrine, de la cyperméthrine, de la cyfluthrine, de la lambda cyhalothrine, de la gamma cyhalothrine, du fenvalérate, de l'alphacyperméthrine, de la pyréthrine et de leurs combinaisons.

10. Régime posologique selon la revendication 6 ou 7, dans lequel le régulateur de croissance des insectes est choisi dans l'ensemble constitué du pyriproxifène, du méthoprène, de la cyromazine, du lufénuron, du diflubenzuron, du fluazuron, du dicyclanil, du fluazuron et de leurs combinaisons.

11. Régime posologique selon la revendication 6, dans lequel l'anthelminitique est choisi dans l'ensemble constitué du lévamisole, du clorsulon, du fipronil, de l'imidacloprid, de la roténone, du flurosilicate de magnésium, du butoxyde de pipéronyle, du spinosyne et de leurs combinaisons.

12. Régime posologique selon la revendication 6, dans lequel le benzimidazole est choisi dans l'ensemble constitué de l'albendazole, de l'oxfenbendazole et du fenbendazole.

13. Régime posologique selon l'une quelconque des revendications 1 à 12, dans lequel l'identificateur unique est un symbole, un code, un marquage, une couleur ou une caractéristique d'un projectile de dosage, l'identificateur unique étant de préférence un signe, un mot, un symbole ou une couleur qui diffère sur chaque projectile de dosage.

14. Régime posologique selon la revendication 13, dans lequel l'identificateur unique consiste en une couleur différente sur chaque projectile de dosage, la couleur étant de préférence incorporée dans la coquille du projectile de dosage ou la couleur étant un colorant contenu à l'intérieur du projectile visible à l'oeil nu.

15. Régime posologique selon l'une quelconque des revendications 1 à 14, comprenant en outre des instructions permettant l'administration de chaque pesticide conformément à l'identificateur unique de chaque projectile de dosage.
